# FASCICULE DE BREVET EUROPEEN

(11) **EP 0 553 024 B1**
(45) Date de publication et mention de la délivrance du brevet: **03.11.1999**
(21) Numéro de dépôt: 93400136.3
(22) Date de dépôt: 20.01.1993
(51) Int. Cl.: A61B 5/14, A61M 5/32

(54) **Dispositif de prélèvement sanguin pour tube à vide d'air**
Blutentnahmevorrichtung von evakuierten Röhrchen
Blood sampling device for a vacuum evacuated sample-tube

(30) Priorité: 22.01.1992 FR 9200680
(43) Date de publication de la demande: 28.07.1993
(73) Titulaire: Barbier, Georges Albert Eugène, F-14000 Caen (FR); Binet, Yvon, F-14112 Bieville (FR); Marquis, Patrick Roger Pierre Paul, F-14000 Caen (FR); Thuaudet, Sylvain, F-14480 Le Fresne Camilly (FR)
(72) Inventeur: Barbier, Georges Albert Eugène, F-14000 Caen (FR); Binet, Yvon, F-14112 Bieville (FR); Marquis, Patrick Roger Pierre Paul, F-14000 Caen (FR); Thuaudet, Sylvain, F-14480 Le Fresne Camilly (FR)
(74) Mandataire: Cabinet HERRBURGER

(56) Documents cités:
- EP-A- 0 272 035
- EP-A- 0 288 879
- EP-A- 0 329 038
- EP-A- 0 363 180
- WO-A-89/04678
- FR-A- 2 653 668
- US-A- 4 774 964
- US-A- 4 850 374
- US-A- 5 030 209
- US-A- 5 086 780

## Description

La présente invention concerne un dispositif de prélèvement sanguin à tube à vide selon le préambule de la 1ère revendication.

Pour effectuer le prélèvement, on engage partiellement le tube dans le manchon en évitant de l'embrocher sur l'aiguille. Puis on pique le patient et on enfonce le tube sur l'aiguille pour perforer son bouchon. Le vide régnant dans le tube crée alors une dépression qui aspire le sang dans le tube. A la fin de ce prélèvement, on extrait le tube du manchon. On peut laisser l'aiguille et le manchon en place pour faire un autre prélèvement sur le même patient. Au cas contraire, on extrait l'aiguille et pour la jeter, il faut y mettre un capuchon. Puis, il faut dévisser l'aiguille et mettre un autre capuchon sur la partie arrière de l'aiguille.

Cette opération nécessite une certaine attention et il arrive que l'utilisateur se pique et risque ainsi une contamination.

Différents types de seringues et de dispositifs de prélèvement sanguin à usage unique sont connus selon la documentation.

Ainsi, le document WO 89/04678 HABLEY décrit un dispositif de prélèvement sanguin selon le préambule de la 1ère revendication, le dispositif comprenant un manchon logeant un disque coulissant, muni d'une aiguille à deux extrémités, l'une pour se piquer dans la veine et l'autre pour perforer le bouchon d'une éprouvette à dépression que l'on embroche après avoir piqué la veine.

Le blocage du disque en position rétractée de l'aiguille ou en position avancée se fait par enfoncement d'un bossage porté par une patte élastique, solidaire du disque à déplacer, dans un logement de dimensions plus grandes que la coulisse à chaque extrémité de celle-ci, cette coulisse étant réalisée dans le manchon. Le dégagement se fait par enfoncement selon un mouvement radial pour escamoter le bossage et permettre le coulissement.

Dans cette réalisation, le coulissement se fait soit manuellement, soit commandé par un ressort.

Le document EP 0 272 035 décrit une seringue avec un corps cylindrique recevant un manchon dont le côté fermé est traversé par une aiguille à deux extrémités, l'une pour se piquer dans la veine, l'autre pour permettre d'embrocher une ampoule contenant le produit à injecter et dans laquelle vient le piston de la seringue. Le mouvement de retrait du piston entraîne celui du manchon avec l'aiguille. Mais seule la position escamotée est légèrement bloquée et cela non de manière définitive.

Dans cette seringue la partie jetable est l'avant avec l'aiguille et la partie arrière avec le piston est réutilisable.

Le document US-A-477 4964 décrit un dispositif de prélèvement sanguin avec un double disque d'une structure très compliquée, et portant de manière mobile l'aiguille de prélèvement.

Enfin, le document EP-A-0 329 038 décrit un dispositif de prélèvement sanguin très proche du premier dispositif analysé ci-dessus.

La présente invention a pour but de remédier à ces inconvénients et se propose de créer un dispositif de prélèvement sanguin à tube à vide à usage unique, de structure simple et assurant une protection particulièrement efficace contre tout risque de blessure par l'aiguille.

A cet effet, l'invention concerne un dispositif de prélèvement à tube à vide, caractérisé selon les moyens exposés dans les revendications.

Le dispositif jetable selon l'invention présente l'avantage d'une structure extrêmement simple, réalisé dans les mêmes matériaux que le dispositif connu et éliminant ainsi tous les problèmes de compatibilité de matériaux. Ce dispositif est beaucoup plus simple que le dispositif, connu, puisque l'aiguille n'a plus à être placée dans un capuchon et un emballage stérile pour être fixée sur l'extrémité avant du manchon recevant le tube à vide. Au contraire, l'ensemble formé par le manchon et l'aiguille est déjà assemblé ce qui réduit le nombre de pièces et par suite le coût. Etant donné les quantités considérables de dispositifs utilisés quotidiennement dans les hôpitaux, ce problème de la réduction du coût combinée à une amélioration considérable de la sécurité de l'utilisation du dispositif constitue un progrès extrêmement important.

En effet, l'utilisation du dispositif selon l'invention est très simple et l'utilisateur ne risque à aucun moment de se piquer, lorsqu'il effectue les manoeuvres de mise en place de l'aiguille et surtout de retrait de l'aiguille d'autant plus que, d'une main, il est obligé de tenir le manchon du dispositif et de l'autre, le tube pour déverrouiller puis tirer le disque de guidage et ainsi l'aiguille dans sa position escamotée.

Ainsi, l'intervention se limite à une action sur des pièces relativement éloignées de la pointe de l'aiguille servant à piquer le patient et l'accrochage entre le disque et le bouchon du tube pour solidariser ces deux parties en rotation, permet le mouvement de déverrouillage et de retrait de l'aiguille en fin d'opération : en tournant le tube, on fait tourner le disque de guidage (accrochage entre le bouchon du tube et les broches), puis en tirant sur le tube, on tire l'aiguille par sa partie arrière grâce au serrement du caoutchouc et au poids de l'aiguille, le dispositif étant retourné pour cette opération (pointe de l'aiguille - côté patient - tournée vers le haut.

L'introduction de l'aiguille et de sa pièce de guidage et de verrouillage au moment de la fabrication peut se faire en jouant sur l'élasticité des parois du manchon pour faire passer la surépaisseur représentée par les ergots jusqu'à ce que ceux-ci apparaissent dans l'ouverture de la coulisse.

Pour ce montage, il est également possible de réaliser un manchon fendu dans sa partie arrière pour le passage des ergots jusque dans la coulisse.

Suivant une autre caractéristique le manchon comporte une deuxième coulisse en position diamétralement opposée par rapport à l'axe du manchon, et présentant une forme symétrique par rapport à l'axe du manchon.

Bien que les coulisses et leur ergot correspondant puissent être prévus dans des positions non symétriques et non diamétralement opposées, il est préférable qu'il en soit ainsi pour répartir mieux les efforts et faciliter les mouvements de glissement.

La présente invention sera décrite ci-après de manière plus détaillée à l'aide des dessins annexés. Ainsi :
- la figure 1 est une vue en coupe schématique d'un manchon de dispositif de prélèvement selon l'invention.
- la figure 2A montre le manchon de prélèvement seul, en coupe axiale.
- la figure 2B est une vue de l'extrémité ouverte du manchon de la figure 2A.
- la figure 3A montre l'aiguille et sa pièce de guidage en vue de côté.
- la figure 3B est une vue de face arrière correspondant à la figure 3A.
- la figure 4 est une vue en coupe d'une variante de réalisation du manchon et de la pièce de guidage de l'aiguille.
- la figure 5 est une vue en coupe axiale du manchon de la figure 4.
- la figure 6 est une coupe d'une variante de réalisation de l'avant du manchon.
- la figure 7 est une coupe d'une autre variante de réaliation de l'avant du manchon.
- Les figures 8A, 8B, 8C montrent en vue de dessus, de face et de côté, le disque muni de l'aiguille selon une variante de réalisation.
- Les figures 9A, 9B montrent en vue de dessus et en coupe axiale, le manchon du dispositif.

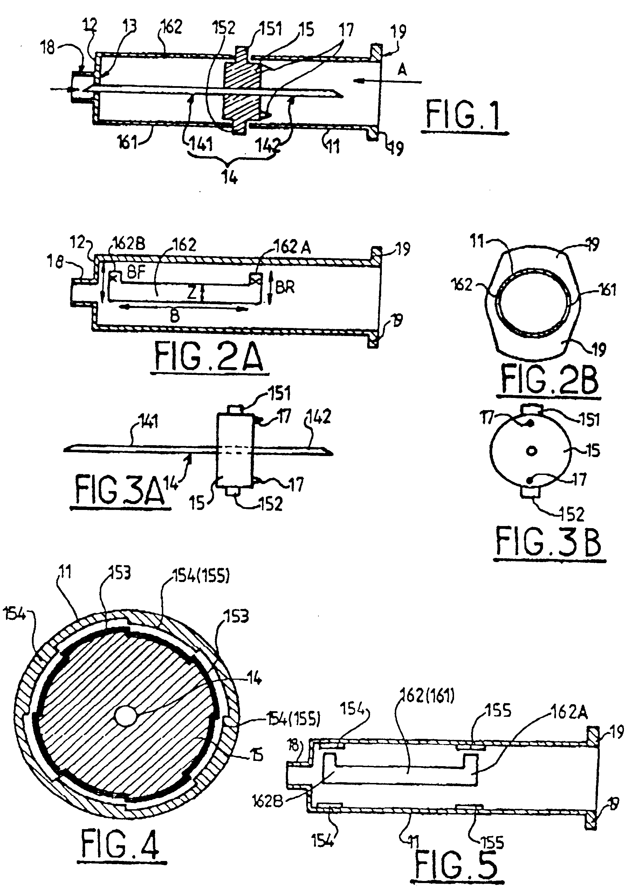

Selon la figure 1, le dispositif de prélèvement sanguin à tube à vide de l'invention, qui n'est représenté que partiellement, se compose d'un manchon 11 dont le fond 12 est muni d'un orifice 13 de passage et de guidage de l'aiguille 14.

L'aiguille 14 comporte deux extrémités 141, 142, la première extrémité 141 servant à piquer le patient et la seconde extrémité 142 servant à perforer le bouchon du tube à vide que l'on introduit dans le manchon de la droite vers la gauche selon la figure 1 (flèche A).

L'aiguille 14 est munie d'un disque de guidage et de verrouillage 15 placé dans le manchon 11 et comportant deux ergots 151, 152 accessibles de l'extérieur du manchon 11 et logés dans des coulisses 161, 162 réalisées dans la paroi du manchon 11. Comme selon la figure 1, les ergots 151, 152 sont diamétralement opposés par rapport à l'axe longitudinal du manchon 11, il en est de même des coulisses 161, 162 qui les reçoivent.

La vue de dessus de la figure 2A montre la forme de la coulisse 162.

En agissant sur les deux ergots 151, 152 on peut ainsi faire passer l'aiguille 14 de la position escamotée représentée à la figure 1 dans la position de sortie, en faisant avancer l'ensemble formé par l'aiguille 14 et le disque de guidage 15 dans sa coulisse.

A chaque extrémité de la coulisse 162, il y a une position de verrouillage 162A, 162B permettant par un mouvement de rotation du disque 15 autour de son axe matérialisé par l'aiguille. Ce mouvement se fait par une rotation transversale suivant l'axe de l'aiguille (double flèche B R) permettant de faire passer l'ergot 151 correspondant à cette coulisse 162, soit dans la position de verrouillage 162A, soit dans la coulisse 162 autorisant le mouvement de coulissement (double flèche B). Le logement de verrouillage 162B à l'autre extrémité de la coulisse 62 a pour fonction de permettre le blocage de l'ergot 151 lorsque l'aiguille 14 est en position d'utilisation.

Dans le cas le plus simple représenté à la figure 2A, la coulisse 162 comporte une partie longitudinale reliant les deux positions de verrouillage. Cette partie longitudinale est parallèle à l'axe du manchon. Il ne s'agit là que d'un mode de réalisation particulier, qui offre l'avantage de la simplicité.

Le dédoublement du prolongement, c'est-à-dire la présence de l'ergot 152 en plus de l'ergot 151 permet une réalisation symétrique, évitant que sous la poussée de translation, le disque 15 ne puisse se gripper.

Le disque 15 comporte sur son côté arrière des moyens d'accrochage tels que des broches 17 ou des barrettes d'accrochage dans lesquelles se pique ou s'accroche le bouchon du tube à vide par son bord pour s'accrocher sur la pièce 15 et permettre le déverrouillage et le retrait après utilisation avec verrouillage complet (mise des ergots 151, 152 dans les encoches 162A), l'aiguille étant à ce moment complètement escamotée dans le tube.

La manoeuvre se fait très simplement : il suffit de tourner le tube de prélèvement sur lui-même en le tenant d'une main, l'autre main tenant le manchon ; puis on tire sur le tube pour rétracter l'aiguille et l'escamoter. Enfin, le disque est verrouillé dans sa position de fin de course et en exerçant un effort plus intense, on détache le tube du disque.

De manière avantageuse, les ergots 151, 152 sont légèrement en saillie par rapport à la surface extérieure du manchon ; l'importance de ce dépassement relève d'un choix ergonomique.

Le manchon se termine à l'avant par une partie tubulaire 18 qui protège l'extrémité de la partie 141 de l'aiguille dépassant de son orifice 13 lorsque l'aiguille est en position escamotée. Cet orifice 13 reste en arrière du biseau de la partie 141 de l'aiguille.

A l'arrière, le manchon comporte deux oreilles de préhension 19 également opposées diamétralement.

Les figures 3A, 3B montrent l'aiguille 14 et la pièce de guidage et de verrouillage 15 sorties du manchon. La figure 3B montre la forme particulière des ergots 151, 152 et la position des broches 17.

Selon les figures 4 et 5, il est intéressant de compléter le verrouillage de la pièce 15 portant l'aiguille 14, dans la première et dans la deuxième position. Pour cela, la pièce 15 comporte des secteurs en saillie 153 (figure 4) destinés à coopérer avec des secteurs 154, 155 en forme de pattes à l'avant et à l'arrière du tube 11 dans des emplacements correspondant aux positions de verrouillage, dépassant par rapport au contour intérieur du manchon 11. Après passage des secteurs 153 entre les secteurs 154, 155, on pivote la pièce 15 comme cela a déjà été décrit ci-dessus et ce pivotement fait passer les secteurs 153 devant les secteurs 154, 155 homologues. Cet accrochage de type baïonnette complète le verrouillage en position d'utilisation. Les secteurs homologues 154, 155 pour le verrouillage en position de travail et en position escamotée apparaissent à la figure 5.

Les secteurs 153 et les secteurs 154, 155 peuvent également coopérer à la manière de cames, directement l'un avec l'autre, dans la mesure où la forme de l'un des secteurs 153 et/ou 154, 155 n'est pas exactement circulaire mais va en s'évasant. Il y a ainsi un effet de coincement ou de came durcissant le mouvement de rotation inverse et conservant ainsi le verrouillage en position soit d'utilisation, soit escamotée.

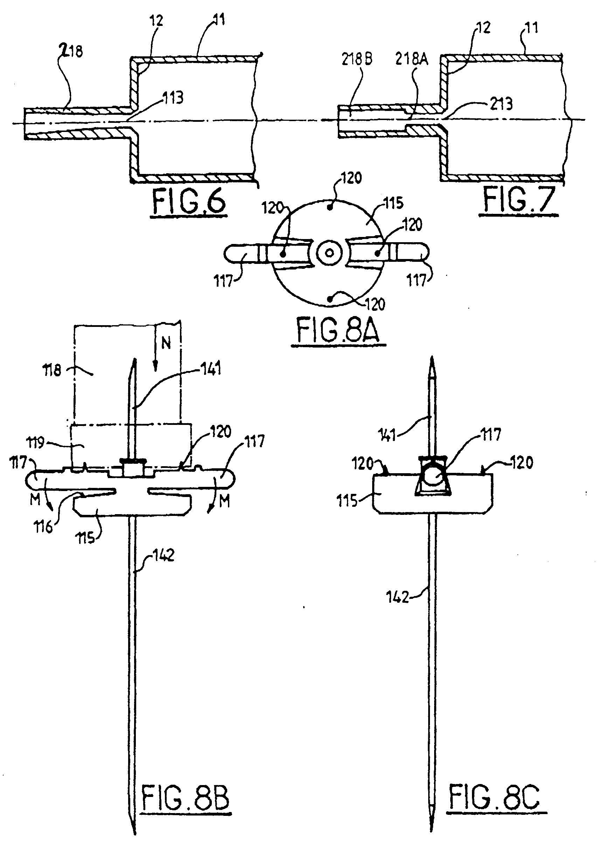

La variante de la paroi avant selon la figure 6 correspond à un manchon 218 allant intérieurement en s'évasant à partir de l'orifice de guidage 113 alors que, dans le mode de réalisation de la figure 7, le manchon comporte un perçage de petit diamètre 218A et un perçage de plus grand diamètre, 218B en avant de l'orifice de guidage 213.

Selon les figures 8A, 8B, 8C, le disque de guidage et de verrouillage 115 portant l'aiguille 14 (141, 142) présente des échancrures 116 pour permettre aux deux languettes 117 constituant les ergots de verrouillage, de pouvoir basculer vers l'arrière (flèches M) lorsqu'une poussée est exercée dans le sens de la flèche N par le tube 118 et son bouchon 119 sur les languettes 117, pour déverrouiller le disque 115 dans sa position avant.

Le dessus du disque 115 et des languettes 117 porte des organes d'accrochage 120 par exemple en forme de pointes sur lesquelles s'embroche le bouchon 119 du tube 118.

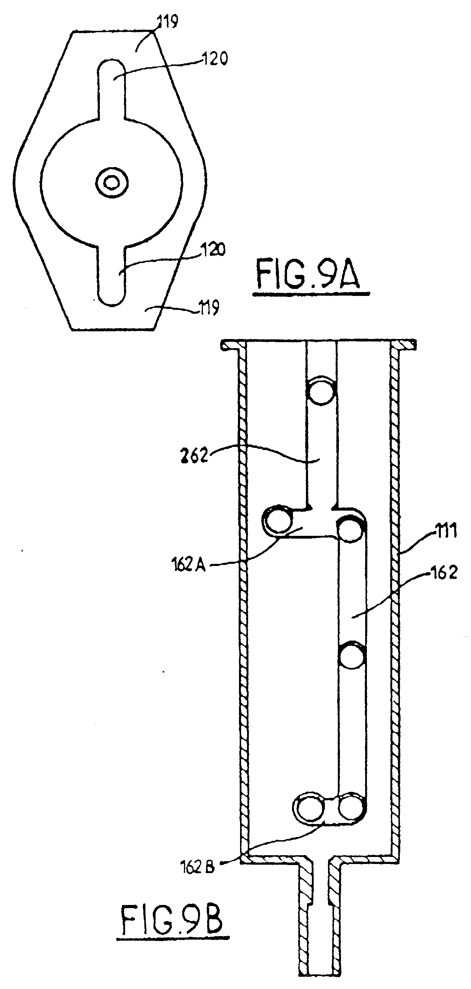

Les figures 9A, 9B montrent une variante 111 du manchon 11 de la figure 1. Selon cette variante, la coulisse 162 est prolongée au-delà de sa tranche 162A par une coulisse d'assemblage 262 qui débouche à l'arrière ouvert du manchon 111 ; pour permettre le passage des ergots 117 (fig. 8B), les oreilles 119 sont également munies de découpes 120. Ainsi pour l'assemblage du dispositif, on glisse le disque et son aiguille, par l'arrière, dans le manchon

De façon générale, les diverses pièces du dispositif présentent entre elles un jeu suffisant pour que les mouvements se fassent aisément tout en permettant un excellent accrochage en position de travail et en position escamotée.

## Revendications

1. Dispositif de prélèvement sanguin comprenant un premier manchon (11) muni d'une aiguille (14) à deux extrémités, dont une première extrémité (141) est destinée à piquer le patient et une seconde extrémité (142), à perforer le bouchon (119) d'un tube à vide (118) qui est placé dans le manchon (11) et qui, après mise en place de la première extrémité (141) dans la veine pour le prélèvement sanguin, est embroché sur l'autre extrémité (142) de l'aiguille (14) pour créer une aspiration de prélèvement sanguin, ladite aiguille (14) pouvant coulisser longitudinalement dans le premier manchon (11) d'une position escamotée verrouillée en retrait dans le premier manchon (11) à une position d'utilisation verrouillée pour laquelle l'aiguille (14) est sortie du premier manchon (11),
* le premier manchon (11) comportant :
- un fond (12) avec un orifice de sortie et de guidage (13) prolongé par un deuxième manchon (18) pour guider et protéger la première extrémité (141) de l'aiguille (14),
- une coulisse de guidage et de verrouillage (162) à deux positions de verrouillage (162A, 162B) correspondant sensiblement au mouvement que doit effectuer l'aiguille (14) pour passer de sa position escamotée à sa position d'utilisation,
* l'aiguille (14) étant munie d'un disque de guidage et de verrouillage (15) placé dans le premier manchon (11) et comportant au moins un ergot (152) logé dans la coulisse (162) et accessible de l'extérieur du premier manchon (11) pour faire passer manuellement l'aiguille (14) de sa position escamotée dans sa position d'utilisation,
dispositif caractérisé en ce que :
- la coulisse (162) se termine à chaque extrémité par un prolongement de verrouillage (162A, 162B) transversal par rapport à la direction de coulissement et qui correspond à chacune des deux positions de verrouillage, pour assurer un verrouillage par un mouvement de rotation du disque de guidage et de verrouillage (15),
- le disque de guidage et de verrouillage (15) comporte des moyens d'accrochage (17) sur sa face arrière, sur lesquels s'embroche le bouchon (10, 10A) du tube à vide (118) pour permettre d'utiliser ce tube (118) comme clé de déverrouillage du disque de guidage (15) et assurer son mouvement de retrait vers la position escamotée de l'aiguille.

2. Dispositif selon la revendication 1,
caractérisé en ce que
le disque (15) et le manchon (11) comportent des moyens complémentaires (153, 154, 155) de blocage par effet de came ou une liaison de type baïonnette.

3. Dispositif selon la revendication 1,
caractérisé en ce que
le disque de guidage et de verrouillage (115) comporte des échancrures (116) et les ergots de verrouillage sont constitués par des languettes (117) qui, sous l'effet d'une poussée exercée sur le tube de prélèvement, peut reculer élastiquement dans les échancrures (116) pour se déverrouiller.

4. Dispositif selon la revendication 1,
caractérisé en ce que
la coulisse (162) est prolongée par une coulisse d'assemblage (262) pour introduire le disque et son aiguille dans le manchon (111).

## Claims

1. Blood sampling device, comprising a first sleeve (11) provided with a needle (14) having two ends, of which a first end (141) is to be inserted in the patient and a second end (142) is to perforate the stopper (119) of a vacuum tube (118) which is placed in the sleeve (11) and which, after the first end (141) has been positioned in the vein in order to remove blood, is skewered onto the other end (142) of the needle (14) to create suction for removing blood, the needle (14) being able to slide longitudinally in the first sleeve (11) from a locked retracted position withdrawn in the first sleeve (11) to a locked use position for which the needle (14) projects from the first sleeve (11),
* the first sleeve (11) comprising:
- a base (12) having an outlet and guide opening (13) which is extended by a second sleeve (18) for guiding and protecting the first end (141) of the needle (14),
- a guide and locking slide (162) having two locking positions (162A, 162B) corresponding substantially to the movement that has to be performed by the needle (14) to pass from its retracted position to its use position,
* the needle (14) being provided with a guide and locking disc (15) placed in the first sleeve (11) and comprising at least one projection (152) which is accommodated in the slide (162) and which is accessible from the outside of the first sleeve (11) in order manually to cause the needle (14) to pass from its retracted position into its use position,
which device is characterised in that:
- the slide (162) is terminated at each end by a locking extension (162A, 162B) which is arranged transversely relative to the sliding direction and which corresponds to each of the two locking positions, in order to provide locking by a rotating movement of the guide and locking disc (15),
- the guide and locking disc (15) comprises, on its rear face, hooking means (17) onto which the stopper (10, 10A) of the vacuum tube (118) is skewered in order to enable the tube (118) to be used as a key for unlocking the guide disc (15) and to ensure that the latter moves backwards towards the retracted position of the needle.

2. Device according to claim 1, characterised in that the disc (15) and the sleeve (11) comprise complementary means (153, 154, 155) for locking by cam effect or a connection of the bayonet type.

3. Device according to claim 1, characterised in that the guide and locking disc (115) comprises recesses (116) and the locking projections are constituted by tongues (117) which, under the effect of thrust exerted on the sampling tube, can move back resiliently into the recesses (116) in order to become unlocked.

4. Device according to claim 1, characterised in that the slide (162) is extended by an assembly slide (262) for introducing the disc and its needle into the sleeve (111).

## Patentansprüche

1. Blutentnahmevorrichtung mit einer ersten mit einer Nadel (14) mit zwei Enden versehenen Hülse (11), wovon das erste Ende (141) zum Einstich in den Patienten und ein zweites Ende (142) zum Durchstechen des Deckels (119) eines Vakuumröhrchens (118) bestimmt ist, welches in der Hülse (11) angeordnet und, nachdem das erste Ende (141) in die Vene zur Blutentnahme eingeführt worden ist, von dem anderen Ende (142) der Nadel (14) durchstochen wird, um eine Blutentnahme unter Ansaugen zu Wege zu bringen, wobei die Nadel (14) in der ersten Hülse (11) in Längsrichtung aus einer verborgenen, verriegelten und in die erste Hülse (11) zurückgezogenen Position in eine verriegelte Betriebsposition vorgeschoben werden kann, in der die Nadel (14) aus der ersten Hülse (11) hervorsteht, wobei
* die erste Hülse (11)
- einen Boden (12) mit einer durch eine zweite Hülse (18) verlängerten Austritts- und Führungsöffnung (13) zur Führung und zum Schutz des ersten Endes (141) der Nadel (14) und
- eine Führungs- und Verriegelungskulisse (162) mit zwei Verriegelungsstellungen (162A, 162B) umfaßt, die im wesentlichen der Verlagerung entspricht, die die Nadel (14) ausführen muß, um aus ihrer verborgenen Stellung in ihre Betriebsstellung zu gelangen,
* die Nadel (14) mit einer Führungs- und Verriegelungsscheibe (15) versehen ist, die in der ersten Hülse (11) angeordnet ist und mindestens einen Vorsprung (152) umfaßt, der in der Kulisse (162) angeordnet und von der Außenseite der ersten Hülse (11) zugänglich ist, um die Nadel (14) von Hand aus ihrer verborgenen Stellung in ihre Betriebsstellung überführen zu können,
dadurch gekennzeichnet,
- daß die Kulisse an jedem Ende in einer Verriegelungsverlängerung (162A, 162B) endet, die quer zu der Kulissenrichtung angeordnet ist und von denen jede einer der beiden Verriegelungsstellungen entspricht, um eine Verriegelung durch eine Drehbewegung der Führungs- und Verriegelungsscheibe (15) zustande zu bringen und
- daß die Führungs- und Verriegelungsscheibe (15) auf ihrer Rückseite Eingriffsmittel (17) trägt, an denen sich der Deckel (10, 10A) des Vakuumröhrchens (118) verhakt, damit das Vakuumröhrchen (118) als Schlüssel für die Entriegelung der Führungs- und Verriegelungsscheibe (15) und zur Herbeiführung der Rückzugsbewegung der Nadel in ihre verborgene Stellung dienen kann.

2. Vorrichtung nach Anspruch 1,
dadurch gekennzeichnet,
daß die Scheibe (15) und die Hülse (11) komplementäre Blockierungsmittel (153, 154, 155) aufweisen, die nach dem Nockeneffekt oder nach Art eines Bajonettverschlusses wirken.

3. Vorrichtung nach Anspruch 1,
dadurch gekennzeichnet,
daß die Führungs- und Verriegelungsscheibe (115) Ausschnitte (116) umfaßt und die Verriegelungsvorsprünge durch Zungen (117) gebildet sind, die unter der Wirkung eines auf das Entnahmeröhrchen ausgeübten Drucks elastisch zur Entriegelung in die Ausnehmungen (116) eintreten können.

4. Vorrichtung nach Anspruch 1,
dadurch gekennzeichnet,
daß die Kulisse (162) durch eine Montagekulisse (262) zur Einführung der Scheibe und ihrer Nadel in die Hülse (111) verlängert ist.
